# EUROPÄISCHE PATENTSCHRIFT

(11) **EP 1 449 503 B1**
(45) Veröffentlichungstag und Bekanntmachung des Hinweises auf die Patenterteilung: **15.09.2004**
(21) Anmeldenummer: 03450041.3
(22) Anmeldetag: 18.02.2003
(51) Int. Cl.: A61F 7/08

(54) **Gerät zur Erzeugung von konzentrischen Temperaturwellen auf der Hautoberfläche**
Device for generating concentric temperature waves on the skin surface
Dispositif pour générer des ondes thermiques concentriques sur la surface de la peau

(43) Veröffentlichungstag der Anmeldung: 25.08.2004
(73) Patentinhaber: Knoglinger, Johann, Dr., 4910 Ried i.l. (AT)
(72) Erfinder: Knoglinger, Johann, Dr., 4910 Ried i.l. (AT)

(56) Entgegenhaltungen:
- AT-B- 407 480
- US-A- 2 198 989
- US-A- 4 261 364
- US-A- 4 586 512
- US-A- 5 628 769
- US-A1- 2002 026 133

## Beschreibung

Nachfolgend beschrieben wird eine Vorrichtung zur Erzeugung konzentrischer Temperaturwellen auf der Haut mit kreis- oder ellipsenförmigen Scheiben und Ringen, Heizelementen, Temperatursensoren und einer elektronischen Regelung und Steuerung. Es kann damit eine Therapie mittels Wärme- und Kältebehandlung durchgeführt werden. Die Erfindung bezieht sich auf das Gebiet der medizinischen Geräte zur physikalischen Therapie.

### Stand der Technik:

Die schmerzlindernde Wirkung von sowohl Wärme als auch Kälte ist seit langem bekannt und gehört zum Standardrepertoire der physikalischen Medizin. Auch die abwechselnde Stimulation der somatischen Nerven mit Wärme- und Kälteimpulsen wird als erleichternd empfunden und es sind schon seit langem Geräte in der Patentliteratur beschrieben, welche diesem Zweck dienen. Im US-Patent 3,168,895 aus dem Jahre 1965 wird ein Gerät dargestellt, welches an zwei getrennten Punkten abwechselnd Wärme- und Kälteimpulse erzeugt und damit Hautpartien stimuliert. Die Applikation von abwechselnd Wärme und Kälte an derselben Hautstelle mittels Thermoelementen basierend auf dem Peltier-Effekt wird im US-Patent 3,207,159 ebenfalls aus dem Jahr 1965 reklamiert.

Im US-Patent 4,585,002 aus dem Jahr 1986 wird ebenfalls die Verwendung von Peltier-Elementen zur wiederholten Temperaturerhöhung und -erniedrigung rund um eine mittlere Temperatur zum Zwecke der lokalen Schmerzbehandlung beschrieben. Die der Haut abgewandte Seite der Thermoelemente verfügt dabei über eine Wärmeaustauschfläche um über natürliche Konvektion überschüssige Wärme oder Kälte abführen zu können. Die Dauer eines Heiz- oder Kühlzyklus bewegt sich zwischen 5 und 60 Sekunden und der Variationsbereich der Temperatur liegt zwischen 15°C und 43°C. Mittels Mikroprozessorsteuerung wird die Stromzufuhr und damit die Temperatur der Peltier-Elemente gesteuert. Im US-Patent 4,741,338 (1988) wird eine an einem Befestigungsgurt angebrachte Reihe von Peltier-Thermoelementen beschrieben, mit denen in einminütigen Zyklen eine Hauterwärmung bzw. -abkühlung gemacht werden kann. Dieser Temperaturwechsel wird als sehr effektiv für die Entfernung von unter der Haut liegendem Fett beschrieben. Die Wärme- bzw. Kälteabfuhr von den hautabgewandten Seiten der Peltier-Elemente wird bei diesem Patent durch Zwangsventilation erreicht.

Die Erzeugung von thermischen Mustern auf der Haut mittels einer Matrix von mikrocomputergesteuerten Peltier-Elementen wird im US-Patent 4,860,748 aus dem Jahre 1989 beschrieben. Unter anderem gelingt es damit, Wärme- und Kältewellen zu erzeugen (allerdings nur als lineare oder ebene Wellen und nicht als kreisförmige, konzentrische Wellen). Die im Patent reklamierten Periodendauem sind größer als 1 Minute. Es werden Peltier-Thermoelemente in der üblichen Bauweise (quadratische oder rechteckige Platten) eingesetzt.

Im japanischen Patent mit der Veröffentlichungsnummer JP0002109557AA (23.04.1990) von T. Miyamae wird eine ausreichende Kühlwirkung für die im thermoelektrischen Behandlungsapparat eingesetzten Peltier-Elemente dadurch erreicht, daß die der Haut abgewandte Fläche der Peltier-Elemente mit kaltem Wasser zwangsgekühlt wird. Außerdem läßt sich mit dieser im Vergleich zur Luftkühlung viel effektiveren Kühlung eine Reduzierung der Dicke des Behandlungsapparates herbeiführen.

Die Aufbringung pulsierender Wärmestimuli zur Erreichung analgesischer Effekte auf der Haut mittels eines geeignet temperierten Luftstroms wird in einer Serie von Patenten von Guibert et al. beschrieben. Beispielhaft seien die folgenden US-Patente angeführt: 4,307,286 (1981); 5,107,832 (1992); 5,190,031 (1993); 5,374,284 (1994); 5,447,530 (1995); 5,580,350 (1996).

Die deutsche Offenlegungsschrift DE 199 05 265 A1 (FOSS) (10.August 2000) zeigt eine Vorrichtung zur Übererwärmung zum Abbau des Übergewichtes durch Anregung des Fettstoffwechsels. Es werden hiezu Radiowellen und Flächensender eingesetzt. Ein wellenartiges Temperaturprofil mit lokal und zeitlich abwechselnder Erwärmung über der Körpertemperatur und Abkühlung unter der Körpertemperatur ist damit nicht machbar.

Die US Patentschrift US 5 601 618 (James) (11.Feber 1997) zeigt eine Wärmevorrichtung für Körpergewebe. Die wärmenden Bereiche sind kreisförmig geformt. Die Vorrichtung wird zur Schmerzbekämpfung eingesetzt. Es ist damit ebenfalls nur eine Erwärmung möglich. Ein wellenartiges, konzentrisches Temperaturprofil mit radial zu einem Mittelpunkt hinlaufenden abwechselnd wärmeren und kälteren Ringen (konzentrische Wärmewelle) ist damit ebenfalls nicht realisierbar.

Die österreichische Patentschrift AT 407 480 B (Macher) (15.August 2000) zeigt ein sehr dünnes kreisförmiges oder ellipsenförmiges Wärmeübertragungselement, welches unter anderem zur Schmerzbekämpfung verwendet wird. Ein Mikroprozessor erzeugt Impulssignale und generiert damit Temperaturwellen. Dem Flächenheizelement ist eine wärmeisolierende Schicht zugeordnet. Weiters ist ein Temperatursensor vorgesehen. Jedoch ist es so, daß es sich hier lediglich um eine lokal fixierte (statische) Temperaturwelle mit einzig Übertemperatur-Charakter handelt. Konzentrisch auf einen Punkt hinoder von einem Punkt weglaufende Temperaturwellen mit zeitlich und lokal variierendem Über- und Untertemperaturprofil (relativ zur Körpertemperatur) sind damit nicht zu verwirklichen.

Die technische Aufgabe der Erzeugung von konzentrisch auf einen Punkt hinoder von einem Punkt weglaufende Temperaturwellen mit zeitlich und lokal variierendem Über- und Untertemperaturprofil (relativ zu einer mittleren Temperatur wie etwa der Körpertemperatur) soll mit der beschriebenen Erfindung gelöst werden. Diese Aufgabe wird dadurch bewältigt, dass zusätzlich zu einer zentralen Scheibe (1a) konzentrische Ringe (1b, 1c) aus dünnem, gut wärmeleitendem Material wie z.B. Silber oder Kupfer vorgesehen sind, dass Spalten aus thermisch isolierendem Material (2) zwischen den Ringen vorgesehen sind, wobei die Heizelemente aus dünnen Heizbahnen (3) bestehen, die auf der Innenseite der Scheibe (1a) bzw. der konzentrischen Ringe (1b, 1c) angebracht sind, wobei sich die Temperatursensoren (4) in Kontakt mit der Scheibe (1a) bzw. den konzentrischen Ringen (1b, 1c) befinden, wobei weiters oberhalb der Scheibe und der Ringe (1a,b,c) und der Heizbahnen (3) eine Materialschicht (5), welche eine definierte Wärmeflussbarrierre darstellt, vorgesehen ist, und weiters oberhalb der Wärmeisolationsschicht (5) ein Kühlmedium (6) vorgesehen ist.

Wie bei der vorhergehenden Beschreibung des Standes der Technik dargestellt, wurden bereits verschiedene Geräte sowohl für die rein lokale Erwärmung und Abkühlung von Hautstellen als auch für die Behandlung benachbarter Hautstellen mit einer Matrix von Peltier-Thermoelementen patentiert. Allen diesen Patenten ist gemeinsam, daß sie die Verwendung von Peltier-Thermoelementen reklamieren. Diese Festlegung auf Peltier-Thermoelemente hat zwar Vorteile in der Einfachheit des Aufbaus eines Thermo-Behandlungsgerätes, bringt jedoch auch gewisse Nachteile mit sich, deren Vermeidung eines der Ziele der vorgeschlagenen Erfindung ist. Die Nachteile der Peltier-Thermoelemente bestehen darin, daß zum einen durch deren typischen Aufbau mit schlecht wärmeleitenden Halbleitermaterialien im Inneren und elektrisch isolierenden Keramikplatten an den Außenflächen die thermische Trägheit so groß ist, daß Temperaturwellen mit kurzen Periodendauem (<5 Sekunden) schwer machbar sind. Zum anderen ist die Form der Peltier-Thermoelemente als quadratische oder rechteckige Platten eine Einschränkung für die Gestaltungsmöglichkeit von Behandlungsgeräten, beispielsweise wenn man konzentrisch von außen nach innen laufende kreisförmige Wärmewellen einsetzen möchte.

Die vorgeschlagene Erfindung betrifft ein medizinisch einsetzbares Gerät zur Erzeugung von konzentrischen, ringförmigen Temperaturwellen auf der Haut, wobei die Periodendauer eines Wärme-/Kältzykluses sogar in der Größenordnung des menschlichen Pulsschlages liegen kann, sodaß damit ein Bio-Feedback auf pulsierende Schmerzen möglich ist. Es werden keine Peltier-Thermoelemente im Inneren des auf der Haut aufliegenden Applikators eingesetzt. Die Anwendung von ringförmigen, zum Zentrum der Applikatorfläche hinlaufenden Temperaturwellen bietet deswegen Vorteile, weil gleichzeitig mit dem Wärmegefühl der Blutdurchfluß in der Haut stimuliert wird und eine Konzentration des hautnahen Blutflusses zum Behandlungszentrum hin gezielt erfolgen kann. Wichtig ist für diese Art der Stimulation der wandernde, ortsveränderliche Charakter der Temperaturwelle. Genauso wie ein Hinlaufen des Temperaturwellen-Bauches zu einem Zentrum kann auch ein Weglaufen von einem Zentrum therapeutisch interessante Stimuli auf das Körpergewebe ausüben, vergleichbar denen einer Massage.

Eine Erzeugung von einzig Übertemperatur-Wellen mittels Heizelementen oder Radiowellen ist nicht allzu schwierig realisierbar. Hingegen stellt es eine größere technische Herausforderung dar, eine konzentrisch auf ein Zentrum hinoder von einem Zentrum weglaufende Über- und Untertemperaturwelle (bezogen auf eine mittlere Temperatur wie beispielsweise der Körpertemperatur) auf der Haut zu erzeugen, und obendrein noch zu versuchen, die Periodendauer bis in den Zeitbereich des menschlichen Pulsschlags zu reduzieren.

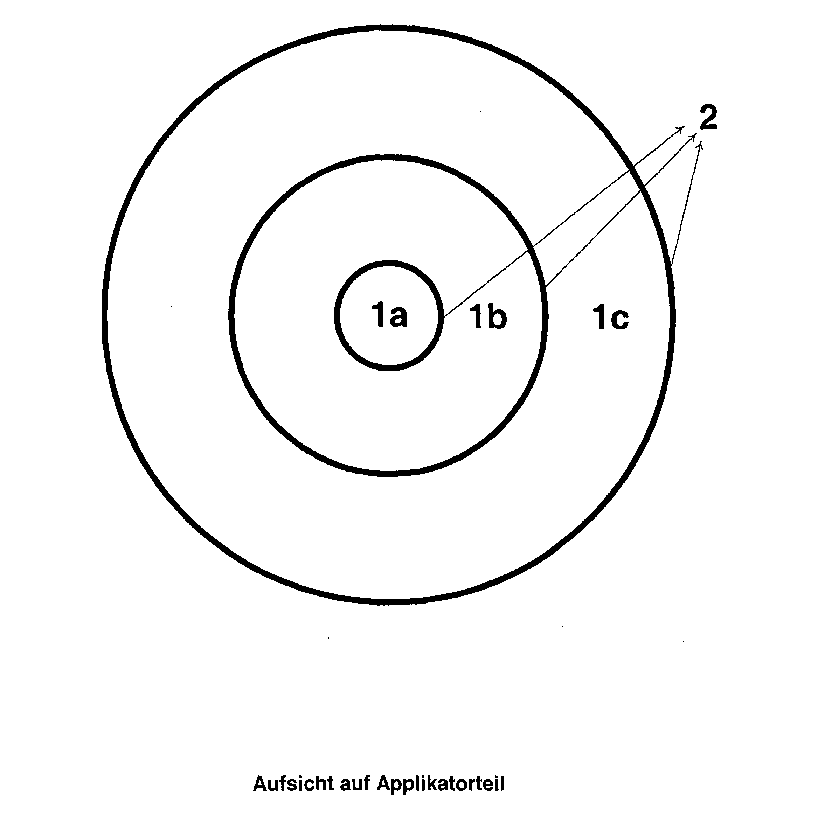

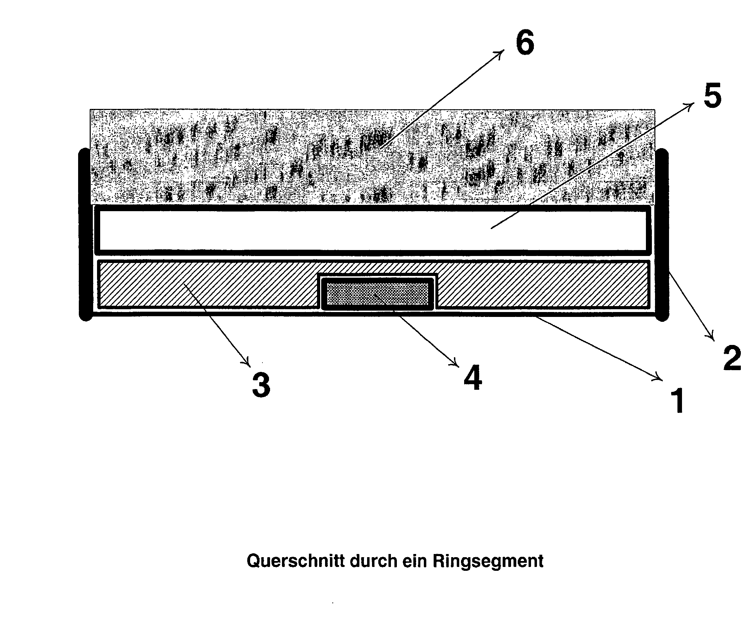

In der Zeichnung 1 ist eine Aufsicht auf die Applikatorfläche in einer möglichen Auslegungsform der Erfindung zu sehen. Diese auf die Haut gehaltene Fläche des Applikators besteht aus mindestens 2 konzentrischen Kreisringen (1b, 1c) und einer zentralen Kreisscheibe (1a), die jeweils aus dünnem, sehr gut wärmeleitendem Material (Silber, Kupfer, Legierungen daraus, ...) gefertigt sind. Die Wandstärke dieser Teile ist dünn (< 1mm) und die Spalten zwischen den einzelnen Segmenten sind mit thermisch isolierendem Material (2) ausgefügt. Die sichtbaren Außenteile der Kreisringe und der Kreisscheibe können aus Gründen der Ästhetik und der Hygiene galvanisiert sein (z.B. vergoldet). Das sehr schnelle Aufheizen der Kreisringe und der zentralen Kreisscheibe gelingt deshalb, weil sehr dünnes, sehr gut wärmeleitendes Material verwendet wird und weil unmittelbar auf der Innenseite dieser Teile dünne Heizbahnen aufgebracht sind.

In der Querschnittszeichnung 2 sind diese zwischen sehr dünnen, elektrisch isolierenden Materialien eingebetteten Heizbahnen (3) zu sehen. In Kontakt mit der zentralen Scheibe und den konzentrischen Ringen befinden sich - zumindest stellenweise - Temperatursensoren (4), beispielsweise Thermowiderstände. Über den Heizbahnen kann sich - je nach Auslegung des Applikators - eine Materialschicht (5) befinden, welche eine definierte Wärmeflußbarriere zwischen dem Heizelement und dem darüber befindlichen Kühlmedium (6) darstellt. In der Zeichnung sind die auf allen elektrischen Bauteilen befindlichen dünnen Elektroisolationsmaterialien (Folien, Lacke, dünne Schläuche, ...) nicht extra eingezeichnet, da diese ohnedies selbstverständlich sind.

Das Kühlmedium wird über eine wärmeisolierte Schlauchverbindung von einem separaten Kühlgerät an den Applikator geliefert. Gleichzeitig ist es nicht ungünstig, wenn das Kühlmedium eine hohe Wärmekapazität besitzt (Wasser, Salzlösungen. glykolische Lösungen oder ähnliches, ...), obwohl auch andere Flüssigkeiten oder Gase geeignet sind (Silikonöle, Thermosöle, Freon ...). Das Hauptprinzip des schnell-temperaturreagiblen Applikators besteht darin, daß in der Aufheizphase vom Heizelement soviel Wärme geliefert wird, daß der in der elektronischen Regelung und Steuerung vorgegebene Temperaturgang an der Applikator-Außenfläche nachgefahren werden kann, jedoch gleichzeitig vermittels der wohldefinierten Wärmewiderstandsbarriere über dem Heizelement das mit konstant niedriger Temperatur angelieferte Kühlmedium nicht zuviel Wärme abtransportiert, um diese Anforderung nicht erfüllen zu können. In der Abkühlphase wird einfach die Heizleistung zurückgefahren oder überhaupt abgeschaltet, sodaß das Heizelement in letzterem Fall als rein Kältedurchleitendes Element wirkt. Das im Applikator befindliche, konstant auf niedriger Temperatur (z.B. 5°C) gehaltene Kühlmedium dient als Kältereservoir in der Abkühlphase des Temperaturwellenzykluses. Die schnelle elektronische Regelung und Steuerung der mit der Haut in Kontakt befindlichen einzelnen Applikatorelemente nutzt die wohlbekannten physikalischen Gesetze des Wärmetransports für Vorausberechnungen aus und erlaubt demgemäß die Ausführung der unter den optimierten Materialbedingungen schnellstmöglichen Temperaturwechsel.

Die in der Erfindung hervorgehobenen Vorteile der Schnelligkeit des Temperaturwechsels (Biofeedback auf pulsierende Schmerzen, ...) und der Gestaltungsfreiheit im Design (konzentrische, kreisförmige Wärme/Kältewellen, ...) ergeben sich aus den Wahlmöglichkeiten, die die vorgeschlagene Ausführungsart gestattet (maximaler Wärmetransport durch hoch-wärmeleitfähige und dünne Materialien, schnelle Aufheizung durch unmittelbar an der Innenfläche der Applikator-Kontaktfläche aufgebrachte großflächige, dünne Heizbahnen sowie durch computergesteuerte Dosierung des Heizstromverlaufs, die Kühlgeschwindigkeit ist hoch durch Verwendung von Kühlmedien mit hoher Wärmekapazität und hoher Wärmeleitfähigkeit, die gestalterische Ausführung als Kreisring, Ellipsenring oder anderen ringförmigen Gebilden ist für Widerstandsheizungen leicht möglich im Gegensatz zu plättchenförmigen Peltier-Elementen, ...).

Das gesamte Gerät besteht an sich aus zwei Teilen, nämlich dem auf die Haut gehaltenen Applikator mit den konzentrischen Kreisringen zur Wärme/Kälteübertragung und dem Steuergerät mit einer schnellen Regelung und Steuerung, einer ästhetisch ansprechenden Prozeßvisualisierung und Programmvorwahl und einem ebenfalls in dieses Steuergerät eingebauten Kühlgerät zur Erzeugung der niedrigen Basistemperatur des Kühlmediums. Der Applikator sowie das Steuergerät sind über eine flexible, wärmeisolierende Leitung verbunden, durch welche sowohl das Kühlmedium als auch die elektrischen Leitungen für die Heizelemente und die Temperaturfühler verlaufen.

Das Steuergerät mit dem darin integrierten Kühlaggregat wird beispielsweise auf einen Tisch gestellt und der Arzt oder der Patient selber nimmt die gewünschten Einstellungen der Parameter auf der Bedienoberfläche vor und kann sie am Bildschirm des Steuergeräts kontrollieren. Der Applikator wird entweder per Hand auf die zu behandelnde Hautstelle gehalten oder mit einem geeigneten Mittel dort festgemacht (Gurt, elastische Binde, Klettverschluß, ...). Auf Knopfdruck startet dann das Gerät und der Arzt oder Patient kann - in diesem Ausführungsbeispiel - am Bildschirm die farblich visualisierten Temperaturverläufe der einzelnen Kontaktzonen des Applikators beobachten. Für Biofeedbackzwecke besitzt das Gerät einen Eingang zur Registrierung eines Biofeedback-Eingangssignals (z.B. Puls, EEG-Signal, ...) und die Möglichkeit mit einem Knopf eine erwünschte Phasenverschiebung zwischen dem Biofeedbacksignal und der Wärmewelle des Applikators einzustellen.

Das Gerät selber ist nicht nur auf die Humanmedizin beschränkt, sondern kann auch in der Tiermedizin eingesetzt werden.

## Patentansprüche

1. Vorrichtung zur Erzeugung konzentrischer, ortsveränderlicher Temperaturwellen auf der Haut mit kreis- oder ellipsenförmigen Scheiben, Heizelementen, Temperatursensoren und einer Elektronik zur Steuerung und Regelung, **dadurch gekennzeichnet, daß** zusätzlich zur Scheibe (1a) konzentrische Ringe (1b, 1c) aus gut wärmeleitendem Material, wie z.B. Silber oder Kupfer, vorgesehen sind, daß Spalten aus thermisch isolierendem Material (2) zwischen den Ringen vorgesehen sind, wobei die Heizelemente aus dünnen Heizbahnen (3) bestehen, die auf der Innenseite der Scheibe (1a) bzw. der konzentrischen Ringe (1b, 1c) angebracht sind, wobei sich Temperatursensoren (4) in Kontakt mit der Scheibe und den Ringen befinden, wobei weiters oberhalb der Ringe, der Scheibe (la,b,c) und der Heizbahnen (3) eine Materialschicht (5), welche eine definierte Wärmeflussbarriere darstellt, vorgesehen ist, und weiters oberhalb der Materialschicht (5) ein Kühlmedium (6) vorgesehen ist.

2. Vorrichtung zur Erzeugung konzentrischer Temperaturwellen auf der Haut nach Anspruch 1, **dadurch gekennzeichnet, daß** ein Kühlmedium aus einem separaten Kühlgerät über eine wärmeisolierende Leitung einem mit der Haut in Kontakt stehendem Applikatorteil zugeführt wird.

3. Vorrichtung zur Erzeugung konzentrischer Temperaturwellen auf der Haut nach einem oder mehreren der vorhergehenden Ansprüche, **dadurch gekennzeichnet, daß** das angestrebte Temperaturprofil mittels konzentrischer elektrischer Heizbahnen und einer Temperatursensorgekoppelten elektronischen Steuerung und Regelung erzeugt wird, wobei die gesteuerte elektrische Heizung der Kühlwirkung des Kühlmediums lokal entgegenwirkt und damit gleichzeitig sowohl über einer wählbaren Mitteltemperatur als auch unter dieser Mitteltemperatur liegende Zonen auf der Applikator-Hautkontaktfläche erreichbar sind.

4. Vorrichtung zur Erzeugung konzentrischer Temperaturwellen auf der Haut nach einem oder mehreren der vorhergehenden Ansprüche, **dadurch gekennzeichnet, daß** Temperaturgänge an einem mit dem Gerät verbundenen Computer vorprogrammierbar sind.

5. Vorrichtung zur Erzeugung konzentrischer Temperaturwellen auf der Haut nach einem oder mehreren der vorhergehenden Ansprüche, **dadurch gekennzeichnet, daß** eine Befestigungsvorrichtung wie beispielsweise ein Gurt oder ein Klettverschluss vorgesehen ist.

6. Vorrichtung zur Erzeugung konzentrischer Temperaturwellen auf der Haut nach einem oder mehreren der vorhergehenden Ansprüche, **dadurch gekennzeichnet, daß** für Biofeedbackzwecke ein Eingang für ein externes Signal, wie es beispielsweise von einem Pulsmessgerät oder einem EEG geliefert wird, vorgesehen ist.

## Claims

1. Device for generating concentric, locally varying temperature waves on the skin using circular or elliptical disks, heating elements, temperature sensors and an electronics for control and adjustment, **characterized by,** that additionally to the disk (1a) concentric rings (1b, 1c) made out of well-heat-conducting material like e.g. silver or copper are foreseen, that gaps made out of thermally insulating material (2) between the rings are foreseen, whereby the heating elements consist of thin heating paths (3) being attached on the inside of the disk (1a) resp. of the concentric rings (1b, 1c), whereby temperature sensors (4) are in contact with the disk and the rings, whereby moreover there is foreseen a material layer (5) above the rings and disk (1a,b,c) and the heating paths (3) acting as a well-defined heat-flow barrier, and where moreover there is foreseen a cooling medium (6) above the material layer (5).

2. Device for generating concentric temperature waves on the skin according to claim 1, **characterized by**, that a cooling medium from a separate cooling device is supplied over a heat-insulated line to the applicator being in contact with the skin.

3. Device for generating concentric temperature waves on the skin according to one or more of the previously cited claims, **characterized by**, that the desired temperature profile is generated by concentric electrical heating paths and a temperature-sensor-coupled electronic control and adjustment, whereby the controlled electrical heating locally acts against the cooling effect of the cooling medium thus allowing on the same time zones on the applicator-skin-contact-area which are above a medium temperature as well as below a medium temperature.

4. Device for generating concentric temperature waves on the skin according to one or more of the previously cited claims, **characterized by**, that the temperature courses can be pre-programmed on a computer which is linked to the device.

5. Device for generating concentric temperature waves on the skin according to one or more of the previously cited claims, **characterized by**, that a fixation device like a belt or a Velcro fastener are foreseen.

6. Device for generating concentric temperature waves on the skin according to one or more of the previously cited claims, **characterized by**, that for reason of bio-feedback there is foreseen an input for an external signal, like it may be delivered - for example - by a pulse-meter or an EEG.

## Revendications

1. Dispositif pour générer des ondes thermiques concentriques et localement variables sur la peau avec des disques circulaires ou elliptiques, des éléments de chauffage, des sondes de température et une électronique pour la commande et l'ajustement, **caractérisé par**, qu'en plus au disque (1a) il sont prévu des anneaux concentriques (1b, 1c) hors de matériel de conduite de la bonne chaleur en tant que par exemple l'argent ou cuivre, qu'entre les anneaux des entailles fait hors du matériel thermoisolant (2) sont prévues, par lequel les éléments de chauffe se composent des chemins minces de chauffage (3) étant attachés sur l'intérieur surface du disque (1a) resp. des anneaux concentriques (1b, 1c), par lequel les sondes de température (4) soient en contact avec le disque et les anneaux, par lequel d'ailleurs une couche matérielle (5) est prévue au-dessus des anneaux et du disque (1a, b, c) et des chemins de chauffage (3) agissant en tant que barrière bien définie d'écoulement de la chaleur, et où d'ailleurs un milieu de refroidissement (6) est prévu au-dessus de la couche matérielle (5).

2. Dispositif pour générer des ondes thermiques concentriques sur la peau selon la revendication 1, **caractérisé par**, qu'un milieu de refroidissement d'un dispositif de refroidissement séparé est conduit a un applicateur étant en contact avec la peau.

3. Dispositif pour générer des ondes thermiques concentriques sur la peau après un ou plusieurs des revendications précédentes, **caractérisé par**, que le profil de température désiré est produit par des chemins de chauffage électriques concentriques et une commande et un ajustement électronique température-sonde-couplé, par lequel le chauffage électrique ajusté localement agisse contre l'effet de refroidissement du milieu de refroidissement permettant de ce fait en même temps sur la surface de contact applicateur-peau des zones qui sont au-dessus d'une température moyenne éligible aussi bien que ci-dessous de cette température moyenne.

4. Dispositif pour générer des ondes thermiques concentriques sur la peau selon un ou plusieurs des revendications précédentes, **caractérisé par**, que les cours de la température peuvent être préprogrammés sur un ordinateur qui est lié au dispositif.

5. Dispositif pour générer des ondes thermiques concentriques sur la peau selon un ou plusieurs des revendications précédentes, **caractérisé par**, qu'un dispositif de fixation est prévu par exemple comme une ceinture ou comme une attache de Velcro.

6. Dispositif pour générer des ondes thermiques concentriques sur la peau selon un ou plusieurs des revendications précédentes, **caractérisé par**, que pour la raison de la bio-rétroaction là est prévu une entrée pour un signal externe, comme lui peut être livré - par exemple - par un impulsion-mètre ou par un EEG.
